# EUROPÄISCHE PATENTANMELDUNG

(11) **EP 1 043 005 A2**
(43) Veröffentlichungstag der Anmeldung: **11.10.2000**
(21) Anmeldenummer: 00105631.6
(22) Anmeldetag: 16.03.2000
(51) Int. Cl.: A61F 13/511, A61F 13/535

(54) **Absorbierender Einwegartikel mit geringer Neigung zur Rücknässung**

(30) Priorität: 08.04.1999 DE 19915716
(71) Anmelder: Christian Heinrich Sandler GmbH & Co. KG, 95126 Schwarzenbach a d Saale (DE)
(72) Erfinder: Höflich, Wolfgang, 95126 Schwarzenbach (DE); Schinkoreit, Wolfram, 95126 Schwarzenbach (DE); Bernhuber, Uwe, 95032 Hof (DE)

(57) **Zusammenfassung**

Beschrieben wird ein absorbierender Artikel welcher aus einer oberen, dem Körper zugewandten flüssigkeitsdurchlässigen Abdeckschicht, aus einer unteren, der Bekleidung des Trägers zugewandten flüssigkeitsundurchlässigen Schicht und einem, sich zwischen diesen Schichten befindlichen mehrschichtigen absorbierenden Kern besteht und welcher zumindest aus einer ersten Schicht, welche aus hydrophilem synthetischen Mikrofaservlies besteht, welches von einem Vliesstoff niedriger Dichte partiell umhüllt ist, und aus einer daran sich anschließenden zweiten Schicht aus hydrophilem synthetischen Mikrofaservlies, welche in Kontakt ist mit einer weiteren Schicht aus hoch wasserspeicherndem Material. Die partielle Umhüllung der ersten Schicht weist an deren Oberseite ausgehend von deren Mittelpunkt eine Öffnung auf, welche rundum vom Vliesstoff niedriger Dichte begrenzt ist. An der Unterseite der ersten Schicht, zu beiden Seiten der Mittelachse in x - Richtung, verbleibt ein nicht umhüllter Bereich, welcher vom Vliesstoff niedriger Dichte begrenzt ist. Die erste Schicht ist an deren Oberseite zumindest an der Stelle der Öffnung mit der flüssigkeitsdurchlässigen Abdeckschicht fest verbunden. Die erste Schicht ist an deren Unterseite zumindest an der Stelle des nicht umhüllten Bereiches mit der zweiten Schicht fest verbunden.

## Beschreibung

Die Erfindung betrifft absorbierende Einwegartikel, wie sie für den Hygienebereich eingesetzt werden, also zum Beispiel für Babywindel, Damenbinden, Slipeinlagen, Erwachseneninkontinenzhilfen ("Erwachsenenwindeln") und dergleichen.

In der folgenden Beschreibung und in den Beispielen wird sich der Einfachheit halber häufig auf die Anwendung des erfindungsgemäßen absorbierenden Artikels als Damenbinde bezogen. Dies ändert aber nichts an der Tatsache, daß auch die weiteren beispielsweise eingangs aufgeführten absorbierenden Artikel im Sinn dieser Erfindung ausgestaltet werden können.
Die Beschreibung des erfindungsgemäßen absorbierenden Artikels befaßt sich im wesentlichen mit dem absorbierenden Teil dieses Artikels, da dieser das Wesen des Artikels ausmacht. Es versteht sich von selbst, daß der erfindungsgemäße absorbierende Artikel mit weiteren sinnvollen Gegenständen, wie z.B. Verschlüssen, Klebestreifen, elastischen Bändern, Beinabschlüssen, Haftsystemen usw. ausgestattet sein kann.

Absorbierende Einwegartikel sind meist schichtweise aufgebaut und bestehen zumindest aus einer körperseitigen, flüssigkeits- und luftdurchlässigen Abdeckschicht, aus einer der Bekleidung zugewandten flüssigkeitsdichten Abdeckschicht und einem zwischen diesen Schichten angeordneten absorbierenden Kern. Die absorbierenden Einwegartikel funktionieren, vereinfacht dargestellt, in der Weise, daß die vom Körper abgegebenen Flüssigkeiten die körperseitige, flüssigkeitsdurchlässige Schicht durchdringen und im absorbierenden Kern aufgenommen und festgehalten werden. Die rückseitige flüssigkeitsundurchlässige Abdeckschicht verhindert, daß Teile der vom Kern aufgenommenen Flüssigkeit den absorbierenden Artikel in Richtung Träger verlassen und dessen Kleidung verschmutzen.

Häufig tritt jedoch der Fall auf, daß eine sogenannte Rücknässung des Hygieneartikels auftritt. Von Rücknässung spricht man bei einem Hygieneprodukt dann, wenn aus dem mit Flüssigkeit beladenem Saugkern unter Druckeinwirkung wieder Flüssigkeit in Richtung Körper abgegeben wird. Ziel moderner Hygieneprodukte ist es, daß die Haut des Benutzers möglichst trocken bleibt, d. h., möglichst wenig Flüssigkeit in Richtung Körper zurückgeführt wird. Es kann zwar ein Großteil der Flüssigkeit im Saugkörper gut durch die in modernen Hygieneartikeln meist vorhandenen superabsorbierenden Partikel festgehalten werden, allerdings fühlt sich dabei der Saugkörper feucht an.

Als Folge der Rücknässung eines Hygieneartikels können Hautirritationen beim Träger wegen des ständig vorhandenen feuchten Mikroklimas, oder aber wegen der beim längerem Tragen des Hygieneartikels auftretenden Zersetzungsprodukte der vom Körper abgegebenen Stoffe, auftreten.

Es ist bekannt, zur Verbesserung der Rücknässungseigenschaft von Hygieneprodukten, sogenannte Flüssigkeitsverteilvliese einzusetzen, wie sie beispielsweise in der DE 19732039 A1 vorgeschlagen werden. Derartige Vliesstoffe gewährleisten eine gute Flüssigkeitsverteilung, in Verbindung mit einer hohen Aufnahmegeschwindigkeit für die Flüssigkeit, so daß große anfallende Flüssigkeitsmengen von der Absorptionsschicht schnell aufgenommen werden können. Zugleich wird die Flüssigkeit in geeigneter Weise auf die Gesamtfläche des absorbierenden Körpers verteilt, so daß ein hoher Anteil der Gesamtkapazität des absorbierenden Kernes für die Flüssigkeitsbindung ausgenutzt werden kann.

Ebenso können, wie in der DE 4447152 A1 beschrieben, absorbierende Mischungen aus Mikrofasern und hydrogelbildenden Substanzen zur Verringerung der Rücknässung eingesetzt werden.

Die DE 19640451 A1 befaßt sich ebenfalls mit einem absorbierenden Artikel, welcher eine geringe Rücknässung aufweist. Der hier beschriebene Artikel ist mehrschichtig und zeigt einen komplizierten Aufbau.

In der WO 97/33546 A1 wird ein absorbierender Artikel mit verbesserten Rücknässungseigenschaften beschrieben, welcher in der Lage ist, durch Schichten mit trichterförmigen Öffnungen die Flüssigkeit sehr schnell dem Kern zuzuführen und diese dort zu binden. Auch der in dieser Schrift vorgeschlagene Artikel hat einen relativ komplizierten Aufbau.

Ebenso wird im US-Patent 4,531,945 ein relativ kompliziert aufgebauter, mehrschichtiger Artikel beschrieben, welcher durch eine zentrale Öffnung eine gute Durchdringung begünstigt.

Das US-Patent 5,810,798 beschreibt die Verbesserung des Rücknässung durch einen mehrlagigen Aufbau unter Verwendung von Materialien mit verschiedenen Sauggeschwindigkeiten.

Es ist das Ziel der vorliegenden Erfindung, einen absorbierenden Artikel mit sehr guten Rücknässungseigenschaften zur Verfügung zu stellen, welcher einen vergleichsweise einfachen Aufbau besitzt und dementsprechend einfach zu fertigen ist.

Die Aufgabe wird gemäß den Merkmalen des Anspruches 1 gelöst. Vorteilhafte Ausgestaltungen der Erfindung werden in den Unteransprüchen beschrieben.

Erläuterung der Zeichnungen:

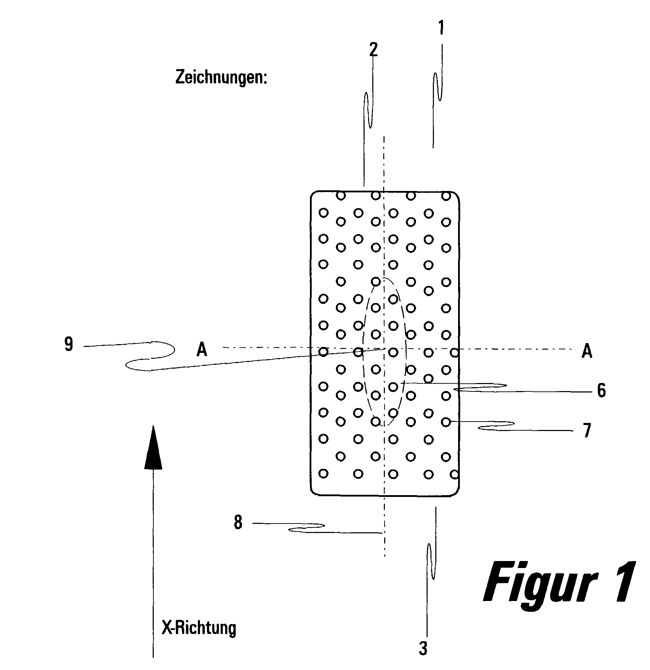

Figur 1 zeigt in Draufsicht die Oberseite einer bevorzugten Ausführungsform des erfindungsgemäßen absorbierenden Artikels.
Figur 2 zeigt den Schnitt entlang der Achse A-A durch den in der Figur 1 gezeigten absorbierenden Artikel.
Figur 3 zeigt eine Detailansicht des erfindungsgemäßen Artikels
Figur 4 zeigt eine weitere Detailansicht des erfindungsgemäßen Artikels
Figur 5 zeigt schematisch die Flüssigkeitsverteilung im erfindungsgemäßen Artikel
Figur 6 zeigt unterschiedliche Ausführungsformen der ersten Kernschicht

Der erfindungsgemäße absorbierende Artikel **1** besteht aus einer oberen, dem Körper zugewandten, flüssigkeitsdurchlässigen Abdeckschicht **10**, aus einer unteren, der Bekleidung des Trägers zugewandten flüssigkeitsundurchlässigen Schicht **11** und einem, sich zwischen diesen Schichten **10, 11** befindlichen, mehrschichtigen absorbierenden Kern **13, 14, 15, 16**.

Die flüssigkeitsdurchlässige Abdeckschicht **10** ist diejenige Schicht des absorbierenden Artikels **1** , welche als erstes mit dem vom Körper abgegebenen Stoffen in Berührung kommt und hat deshalb die Aufgabe, zumindest die flüssigen Bestandteile dieser Stoffe aufzunehmen und diese ins Innere des absorbierenden Artikels **1** passieren zu lassen. Die flüssigkeitsdurchlässige Abdeckschicht **10** hat deshalb eine luft- und flüssigkeitsdurchlässige, poröse Struktur.

Als flüssigkeitsdurchlässige Abdeckschicht **10** kommen unterschiedliche Materialien in Frage. Für den erfindungsgemäßen absorbierenden Artikel **1** werden bevorzugt Vliesstoffe eingesetzt. Es können auch perforierte Folien Verwendung finden, welche zusätzlich mit einem Vliesstoff verbunden sein können. Eine geeignete Folienabdeckung wird beispielsweise in der WO 96/26697 A1 beschrieben.

In einer besonders bevorzugten Ausführungsform besteht die flüssigkeitsdurchlässige Abdeckschicht **10** des erfindungsgemäßen absorbierenden Artikels **1** aus einem perforierten Vliesstoff, wie er beispielsweise in der EP 164740 B1 beschrieben wird. In der am meisten bevorzugten Ausführungsform besteht die flüssigkeitsdurchlässige Abdeckschicht **10** aus einem hydrophoben perforierten Vliesstoff, da sich gezeigt hat, daß dieser in Verbindung mit dem erfindungsgemäßen Aufbau des absorbierenden Artikels **1** die besten Ergebnisse bezüglich der Eindringgeschwindigkeit der Körperflüssigkeit und Verhinderung der Rücknässung bringt.

Als Vliesstoffe kommen kardierte, thermisch verfestigte oder bindemittelverfestigte Vliesstoffe, weiterhin Spinnvliesstoffe, Meltblownvliesstoffe sowie Kombinationen daraus oder auch wasserstrahlverfestigte Vliesstoffe zum Einsatz (Literatur: Lünenschloß/Albrecht: 'Vliesstoffe"; Georg Thieme Verlag, Stuttgart, New York).

Die flüssigkeitsdurchlässige Abdeckschicht **10** hat üblicherweise eine Flächenmasse von 8 bis 100 g/qm, bevorzugt 12 bis 50 g/qm. Im Falle der Verwendung von Vliesstoffen bestehen die Rohmaterialien aus Polyester, Polyamid, Polyacrylnitril, Viskose oder Naturfasern oder aus Mischungen der genannten Rohstoffe. Bevorzugt bestehen die für die flüssigkeitsdurchlässige Abdeckschicht **10** verwendeten Rohstoffe aus Polyolefinen wie Polyethylen oder Polypropylen. Auch Vliesstoffe aus Bikomponentenfasern, deren Kern beispielsweise aus Polypropylen und deren Mantel beispielsweise aus Polyethylen besteht, sind geeignet.

Die bekleidungsseitige flüssigkeitsundurchlässige Schicht **11** hat die Aufgabe, die unmittelbar an den absorbierenden Artikel **1** anschließende Bekleidung des Trägers und auch die darüberliegenden Schichten der Bekleidung des Trägers vor Verschmutzung zu schützen, welche aus dem absorbierenden Kern austreten könnte. In einer bevorzugten Ausführungsform besteht die flüssigkeitsundurchlässige Schicht **11** aus einem wasserdichten und luftdichten Material, wie beispielsweise einer Kunststoffolie. In einer weiteren bevorzugten Ausführungsform kann diese Kunststoffolie zur Verbesserung der textilen Anmutung mit einem Vliesstoff kaschiert sein, wie beispielsweise in der WO 97/00056 A1 vorgeschlagen.

In einer weiteren bevorzugten Ausführungsform ist die flüssigkeitsundurchlässige Schicht **11** wasserdicht, aber wasserdampfdurchlässig ausgeführt. So wird beispielsweise in der WO 97/16148 A1 eine derartige Abdeckung vorgeschlagen, welche aus zumindest einer Spinnvlieslage und aus zwei Meltblownlagen besteht. Auch einfache Ausführungen eines Spinnvlies/Meltblown-Verbundvlieses als flüssigkeitsundurchlässige Schicht **11** sind geeignet. In einer weiteren bevorzugten Ausführungsform besteht die flüssigkeitsundurchlässige Schicht **11** aus einer wasserdichten, aber wasserdampfdurchlässigen Polyethylenfolie und in einer weiteren bevorzugten Ausführungsform aus einer Kombination aus vorgenannter wasserdampfdurchlässiger und wasserdichter Folie in Verbindung mit einem Vliesstoff. In der am meisten bevorzugten Ausführungsform besteht die flüssigkeitsundurchlässige Schicht **11** aus einer Kombination zwischen einem Vliesstoff und einem Papier. Eine derartige Schicht wird in der DE 19912044 A1 beschrieben.

Der absorbierende Kern **13, 14, 15, 16** des erfindungsgemäßen absorbierenden Artikels **1** ist mehrschichtig aufgebaut. Die erste Kernschicht **13** besteht aus einem hydrophilen, synthetischen Mikrofaservlies, welches von einer Umhüllung **15** aus einem Vliesstoff niedriger Dichte "partiell umhüllt" ist. Daran anschließend folgt eine zweite Kernschicht **14** aus einem hydrophilen, synthetischen Mikrofaservlies, welches mit dem der ersten Kernschicht **13** identisch sein kann, oder aber auch sich von dieser Schicht zum Beispiel in der Flächenmasse unterscheiden kann. Darauf folgt eine weitere Kernschicht **16** aus einem hoch wasserspeichernden Material.

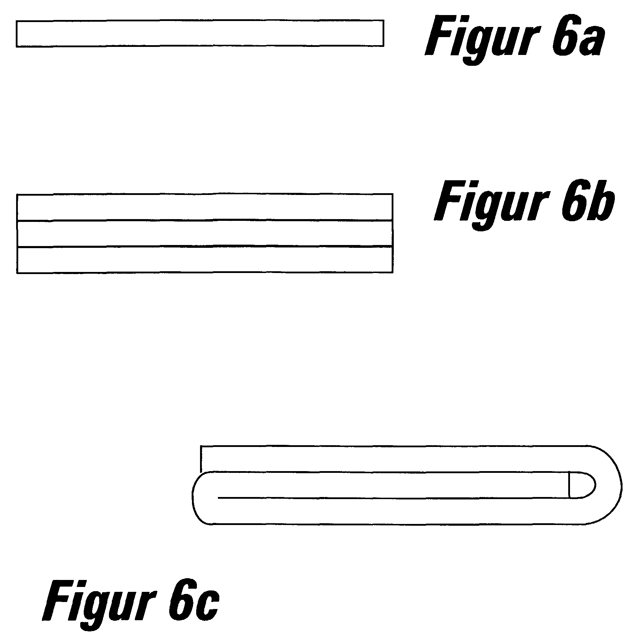

Das hydrophile, synthetische Mikrofaservlies der ersten Kernschicht **13** besteht bevorzugt aus einem Polypropylen-Meltblown-Vliesstoff, welcher über den Zusatz eines benetzenden Mittels hydrophiliert ist. Dies kann entweder dadurch geschehen, daß bei der Vliesstoffertigung der Granulatschmelze ein hydrophilierendes Mittel zugegeben wird, oder aber dadurch, daß der fertige Vliesstoff mittels einer Nachbehandlung mit einem Tensid versehen wird. Möglich ist auch eine Kombination der beiden Verfahren. Hydrophile Meltblownvliesstoffe werden beispielsweise in der US-Patentschrift 4, 307, 143 oder in der US-Patentschrift 4, 578, 414 beschrieben. Der Vliesstoff der ersten Kernschicht **13** weist eine Flächenmasse von 10 bis 100 g/qm auf. Bevorzugt weist der Vliesstoff eine Flächenmasse von 30 bis 70 g/qm auf. Der Vliesstoff kann in planer Form (**Figur 6a)** in den absorbierenden Artikel **1** integriert sein, er kann aber auch in mehrschichtiger Form (**Figur 6b)** vorliegen, wovon die gefaltete Form **(Figur 6c)** eine bevorzugte Ausführungform darstellt. Diese erste Kernschicht **13** wird von einer Umhüllung **15** aus einem Vliesstoff niedriger Dichte partiell umgeben.

### Die Bezeichnung "partielle Umhüllung" umfaßt zwei Teilaspekte:

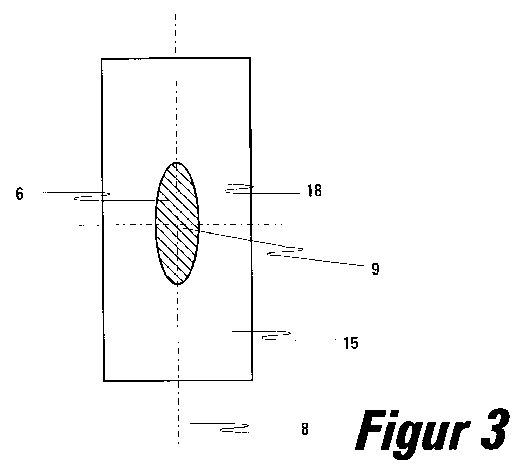

Der erste Teilaspekt bedeutet, daß an der Oberseite der ersten Kernschicht **13 (Figur 3)** diese Umhüllung **15** eine Öffnung **6** ausgehend vom Schnittpunkt der Längs- und Querachse **9** des absorbierenden Artikels **1** aufweist, an der die Umhüllung **15** unterbrochen ist. Diese Öffnung **6** ist so dimensioniert, daß sie maximal 70 % der Breite des absorbierenden Artikels **1** und maximal 50 % der Länge des absorbierenden Artikels **1** umfaßt. Die Form dieser Öffnung **6** ist beliebig. Bevorzugt wird eine zur Längsmittelachse **8** des absorbierenden Artikels **1** symmetrische, runde, ovale, nierenförmige oder lanzettförmige Form. Die Ränder **18** der Öffnung **6** werden von der Umhüllung **15** begrenzt.

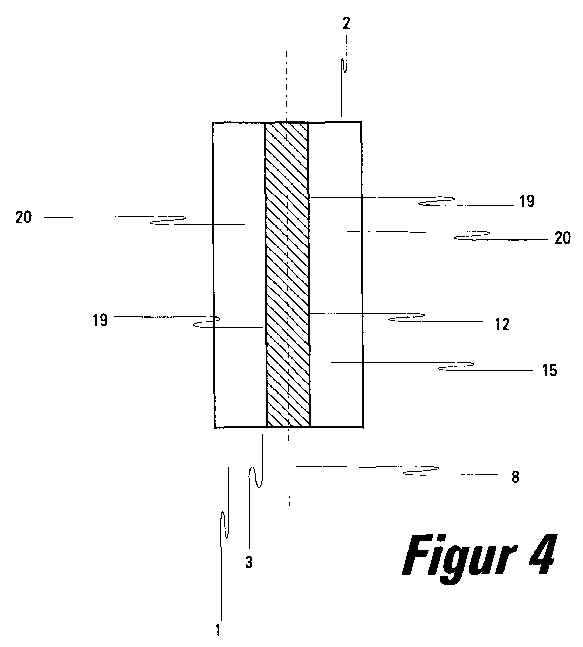

Der zweite Teilaspekt bedeutet, daß sich auf der Unterseite **(Figur 4)** der ersten Kernschicht **13** der nicht umhüllte Bereich **12** zu gleichen Teilen links und rechts der Längsmittelachse **8** des absorbierenden Artikels **1** in Längsrichtung durchgehend erstreckt. Es ist vorteilhaft, wenn die Breite des nicht umhüllten Bereiches **12** 70 % der Gesamtbreite des absorbierenden Artikels **1** nicht überschreitet. Der nicht umhüllte Bereich **12** ist im wesentlichen durchgehend von der vorderen Stirnseite **2** des absorbierenden Artikels **1** zur hinteren Stirnseite **3** des absorbierenden Artikels **1** und wird an den Seiten **20** von der Umhüllung **15** an im Längsbereich **19** begrenzt.

Unter "umhüllt' im Sinne dieser Erfindung ist eine Bedeckung der Oberseite, der Unterseite und der seitlichen Längsränder der ersten Kernschicht **13** zu verstehen. Selbstverständlich umfaßt dieser Begriff aber auch eine Ausführungsform, in der mindestens die Oberseite und die Unterseite der ersten Kernschicht **13** in der vorher beschriebenen Form bedeckt sind, nicht aber die seitlichen Längsränder der ersten Kernschicht **13**. Diese Ausführungsform kann beispielsweise durch ein Einlegen der Umhüllung **15** in Form von mehreren Streifen geschehen, welche dann die Oberseite und die Unterseite, nicht aber die seitlichen Längsränder der ersten Kernschicht **13** in der vorher beschriebenen Form bedecken.

Bei der Umhüllung **15** handelt es sich um einen Vliesstoff aus synthetischen Fasern, wie z. B. aus Polypropylenfasern, aus Polyesterfasern, aus Polyester/Copolyester-Bikomponentenfasern, aus Polyester/Polyethylen-Bikomponenten-fasern oder aus Mischungen der vorgenannten Fasern. Die Dichte dieses Vliesstoffes beträgt, gemessen in Anlehnung an DIN 53855, Teil 2 (Bestimmung der Vliesstoffdicke bei niederen Vorlasten), 10 bis 50 kg/cbm, bevorzugt 15 bis 30 kg/cbm bei einer Flächenmasse von 25 bis 70 g/qm. Ein derartiger Vliesstoff ist im wesentlichen aus der DE 29723320 U1 bekannt, wobei bemerkt werden muß, daß in der vorliegenden Erfindung der besagte Vliesstoff nicht als Flüssigkeitsverteilvlies eingesetzt ist, sondern als eine Art "Rückschlagsbremse", um zu verhindern, daß Flüssigkeit aus dem absorbierenden Kern in Richtung Körper des Trägers abgegeben wird. Die Tatsache, daß es sich bei dem vorgenannten Einsatz nicht um ein Flüssigkeitsverteilvlies handelt, wird dadurch belegt, daß gerade diejenige Stelle, des absorbierenden Artikels **1**, welche mit der größten Flüssigkeitsmenge in Berührung kommt, durch die vorstehend beschriebene Öffnung **6** an der Oberseite der ersten Kernschicht **13** nicht mit der Umhüllung **15** belegt ist und somit keine Flüssigkeitsverteilfunktion ausgeführt werden kann.

Es ist nämlich wesentlich für den erfindungsgemäßen absorbierenden Artikel **1**, daß die Umhüllung der ersten Kernschicht **13** partiell erfolgt, wobei den nicht umhüllten Bereichen **6, 12** eine Schlüsselfunktion in der Flüssigkeitsleitung zukommt. So wird an dem nicht umhüllten Bereich **6** die Flüssigkeit in das Innere des absorbierenden Artikels geleitet, wogegen sie an den umhüllten Stellen am Rückfluß gehindert wird. Der nicht umhüllte Bereich **12** dient zur Weiterleitung der Flüssigkeit in die zweite Kernschicht **14** und die weitere Kernschicht **16**.

Die Umhüllung **15** kann mit der ersten Kernschicht **13** z. B. durch Hotmeltkleber fest verbunden sein, sie kann aber auch lose um die erste Kernschicht **13** gelegt sein, um nur eine lockere Verbindung zur ersten Kernschicht **13** einzugehen.

Die zweite Kernschicht **14** des erfindungsgemäßen absorbierenden Artikels **1** besteht wie die erste Kernschicht **13** aus einem hydrophilen, synthetischen Mikrofaservlies, bevorzugt aus einem hydrophilen Polypropylen-Meltblown-Vliesstoff. Diese zweite Kernschicht **14** kann in den absorbierenden Artikel **1** plan eingelegt sein, sie kann aber auch bei Bedarf zusammengefaltet sein, so daß sich mehrere Lagen übereinander ergeben. Die zweite Kernschicht **14** kann die gleichen Flächenmassen aufweisen wie die erste Kernschicht **13**, sie kann von dieser aber auch abweichende Flächenmassen besitzen. Das hydrophile, synthetische Mikrofaservlies der zweiten Kernschicht **14** weist eine Flächenmasse von 10 bis 100 g/qm, bevorzugt von 30 bis 70 g/qm, auf. Auch diese kann, wie in Figur 6 a bis 6 c dargestellt, ein- oder mehrlagig ausgeführt sein.

Der erfindungsgemäße absorbierende Artikel **1** besitzt eine, sich an die zweite Kernschicht **14** anschließende weitere Kernschicht **16** aus hoch wasserspeicherndem Material. Dieses Material besteht im wesentlichen aus Cellulose-Fasern, welche beispielsweise zur Verfestigung weitere Fasern, bevorzugt Polyolefin-Fasern, am meisten bevorzugt Polypropylen/Polyethylen-Bikomponentenfasern, enthalten können. Diese weitere Kernschicht **16** aus wasserspeichernden Material liegt bevorzugter Maßen in Form eines Tissue oder in Form eines Airlaid-Vliesstoffes vor.

Die weitere Kernschicht **16** kann alternativ aus einer Schicht aus superabsorbierenden Partikeln oder aus superabsorbierenden Fasern bestehen. Derartige Materialien bestehen häufig aus leicht vernetzter Polyacrylsäure und sind dem Fachmann geläufig, weshalb sie hier nicht näher erläutert werden müssen.

In einer bevorzugten Ausführungsform besteht die weitere Kernschicht **16** aus hoch wasserspeichernden Material wie Tissue oder Airlaid-Vliesstoff in Verbindung mit superabsorbierenden Partikeln und/oder superabsorbierenden Fasern.

Die dem Körper zugewandte flüssigkeitsdurchlässige Abdeckschicht **10** ist zumindest an der Stelle der Öffnung **6** der Umhüllung mit der Oberseite der ersten Kernschicht **13** mit dieser fest verbunden. Dies ist notwendig, um eine Flüssigkeitsleitung von der flüssigkeitsdurchlässigen Abdeckschicht **10** in die erste Kernschicht **13** zu gewährleisten. Es widerspricht allerdings nicht dem Sinn der Erfindung, daß die Oberseite des absorbierenden Artikels ganzflächig, d. h.im Bereich der Öffnung **6** und am oberseitigen Teil der Umhüllung **15**, mit der flüssigkeitsdurchlässigen Abdeckschicht **10** fest verbunden ist. Für die Verbindung stehen dem Fachmann bekannte Methoden wie z. B. spiralförmiger oder gesprühter Hotmeltkleberauftrag zur Verfügung.

Die erste Kernschicht **13** ist weiterhin zumindest an ihrer Unterseite im nicht umhüllten Bereiche **12** mit der zweiten Kernschicht **14** fest verbunden. Hierbei entsteht im nicht umhüllten Bereich **12** entlang der Längsmittelachse **8** eine Zone, in der eine Flüssigkeitsleitung von der ersten Kernschicht **13** in die zweite Kernschicht **14** gewährleistet ist , während in den Randbereichen **20, 21** also an den Stellen, an denen die Umhüllung vorliegt, kein Flüssigkeitstransport von der ersten Kernschicht **13** in die zweite Kernschicht **14** stattfinden kann, genau so wenig, wie ein Flüssigkeitstransport von der zweiten Kernschicht **14** zurück in die erste Kernschicht **13** und weiter zurück an den Körper des Trägers.

Durch diese Maßnahme ist gewährleistet, daß die in die zweite Kernschicht **14**, in die weitere Kernschicht **16** sowie in die Randbereiche der zweiten Kernschicht **14** eingedrungene Flüssigkeit dort festgehalten wird und zu keiner unerwünschten Rücknässung führt.

Die feste Verbindung zwischen der ersten Kernschicht **13** und der zweiten Kernschicht **14** kann nur an der Stelle des nicht umhüllten Bereiches **12** , aber auch über die ganze Fläche der Unterseite der ersten Kernschicht **13** , also auch im Bereich der Umhüllung **15**, vorhanden sein. Die Verbindung geschieht durch dem Fachmann geläufige Verfahren wie z. B. eine Verklebung durch spiralförmig aufgetragene oder versprühte Hotmeltkleber.

Ein wesentlicher Aspekt der Erfindung ist die Ausbildung eines Dichtegradienten, welcher bei der Umhüllung **15** an den Übergängen **17** zur Öffnung **6**, an der Oberseite der ersten Kernschicht **13** und zum nicht umhüllten Bereich **12** an der Unterseite der ersten Kernschicht **13** auftritt. Dieser Dichtegradient entsteht, weil in den besagten Randbereichen **18,** die Umhüllung **15,** die aus dem Vliesstoff niederer Dichte besteht, durch die flüssigkeitsdurchlässige Abdeckschicht **10** komprimiert wird. Das gleiche geschieht durch die zweite Kernschicht **14** welche die Ränder **19** der Umhüllung **15** komprimiert. Hierbei stellt sich an den Bereichen **17** eine höhere Dichte an der Umhüllung **15** ein, als in deren übrigen Bereichen. Dieser Dichtegradient trägt dazu bei, daß sich die aufzunehmende Körperflüssigkeit durch auftretende Kapillarkräfte in die Breite des absorbierenden Artikels **1** verteilt und, einmal dort angelangt, am Rückfluß in Richtung Körper gehindert wird.

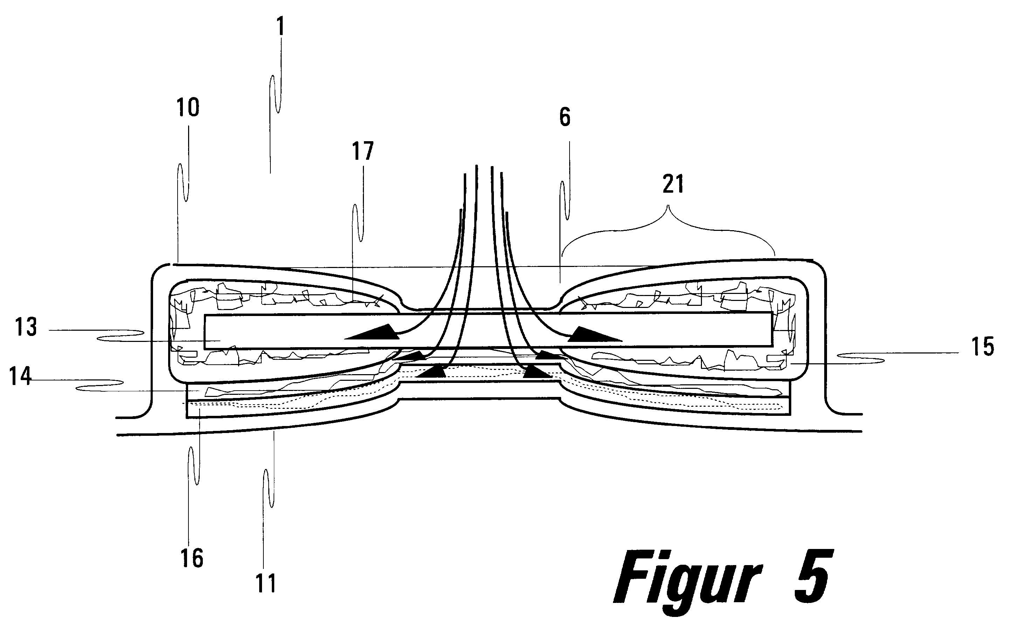

Die **Figur 5** zeigt schematisch durch die Pfeile gekennzeichnet den Flüssigkeitsfluß in den und innerhalb des absorbierenden Artikels **1**. Die meiste Flüssigkeit tritt demnach körperseitig in der Nähe der Öffnung **6** auf, durchdringt an der Stelle der Öffnung **6** die flüssigkeitsdurchlässige Abdeckschicht **10**, welche bevorzugt Öffnungen **7** enthält und tritt in den absorbierenden Artikel **1** ein.

Aufgrund des hydrophilen Charakters des synthetischen Mikrofaservlieses der ersten Kernschicht **13** und durch den engen Kontakt mit der flüssigkeitsdurchlässigen Abdeckschicht **10** in Folge deren festen Verbindung mit der ersten Kernschicht **13**, wird die Flüssigkeit sehr schnell aufgenommen und zumindest ein Teil der anfallenden Flüssigkeit bereits innerhalb der ersten Kernschicht **13** in der Breite verteilt. Der größte Teil der in der Breite verteilten Flüssigkeit gelangt dabei in diejenigen Bereiche **21**, welche von der Umhüllung **15** mit dem Vliesstoff niedriger Dichte bedeckt sind. Aufgrund der niedrigen Dichte des Vliesstoffes und aufgrund dessen Dicke, treten innerhalb des Vliesstoffes, mit Ausnahme der Stelle des Dichtegradienten **17**, kaum Kapillarkräfte auf und demzufolge fließt die Flüssigkeit nicht zurück in Richtung flüssigkeitsdurchlässige Abdeckschicht **10**.

Der andere Teil der anfallenden Flüssigkeit gelangt im nicht umhüllten Bereich **12** an der Unterseite der ersten Kernschicht **13** , welcher zur zweiten Kernschicht **14** aufgrund der festen Verbindung zu dieser hohe Kapillarkräfte ausbildet, in die zweite Kernschicht **14**, welche ebenfalls aus einem synthetischen Mikrofaservlieses besteht. Ein großer Teil dieser Flüssigkeitsmenge wird innerhalb der zweite Kernschicht **14** in die Breite verteilt und an der Stelle der Bereiche **21**, welche von der Umhüllung **15** bedeckt sind, am Rückfluß in die erste Kernschicht **13** und zur körperseitigen Abdeckung **10** gehindert.

Der restliche Teil der Flüssigkeitsmenge gelangt von der zweiten Kernschicht **14** in die weitere Kernschicht **16** welche aus hoch flüssigkeitsbindendem Material besteht und wird dort gebunden.

Aufgrund dieser Konstruktionsweise wird der Rücknässungstendenz von Hygieneartikeln in optimaler Weise entgegengewirkt.

### Ausführungsbeispiel:

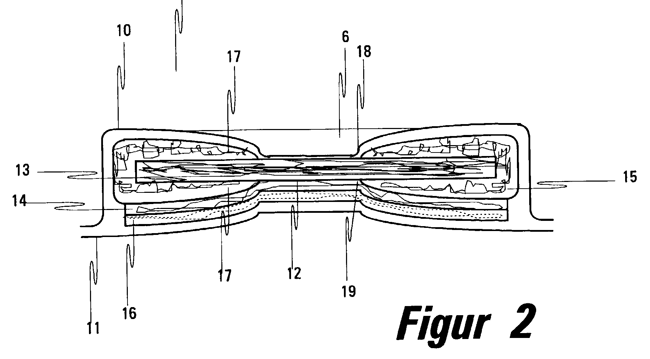

### Eine Damenbinde wird analog der Darstellung der Figur 2 aufgebaut:

Die flüssigkeitsdurchlässige Abdeckschicht **10** besteht aus einem gekrempelten hydrophoben Polypropylen-Thermobondingvliesstoff mit einer Flächenmasse von 24 g/m², welcher Perforationen bei einer offenen Fläche von ca. 21 % aufweist.

Die erste Kernschicht **13** und die zweite Kernschicht **14** bestehen je aus einem Polypropylen-Meltblown-Vliesstoff mit einer Flächenmasse von je 60 g/m², welcher mitttels eines Tensides hydrophiliert wurde.

Die Umhüllung **15** besteht aus einem heißluftverfestigten gekrempelten Vliesstoff, bestehend aus einer Polyethylen/Polyester-Mantel/Kern-Bikomponentenfaser des Fasertiters von 3,3 dtex, mit einer Flächenmasse von 30 g/m² und einer Dicke gemessen nach DIN 53855 T2 von 1,2 mm, was einer Dichte von 25 kg/m³ entspricht.

Die weitere Kernschicht **16** besteht aus einem Tissue-Papier mit einer Flächenmasse von 70 g/m²

Als flüssigkeitsundurchlässige Schicht **11** wurde eine Polyethylenfolie einer Stärke von 18 µ verwendet.

Die so gefertigte Damenbinde hatte eine rechteckige Form und eine Länge von 180 mm und eine Breite von 65 mm. Die Umhüllung **15** wurde so um die erste Kernschicht **13** gelegt, daß unterseitig ein sich in X-Richtung erstreckender nicht bedeckter Bereich **12** mit einer Breite von 15 mm entstand. Oberseitig wurde durch eine Aussparung in der Umhüllung **15** eine lanzettförmige Öffnung **6** mit einer Länge von 40 mm und einer Breite von 25 mm, mittig zum Schnittpunkt **9** der Längs- und der Querachse der Oberseite angeordnet.

Die flüssigkeitsdurchlässige Abdeckschicht **10** wurde im Bereich der Öffnung **6** mittels 1,5 g/m² aufgesprühten Hot-melt-Klebers an der ersten Kernschicht **13** befestigt.
Die erste Kernschicht **13** wurde an der Stelle des unteren nicht umhüllten Bereiches **12** an der zweiten Kernschicht **14** ebenfalls mit mittels 1,5 g/m² aufgesprühten Hotmelt-Klebers befestigt.

Die weitere Kernschicht **16** wurde unter die zweite Kernschicht **14** lose eingelegt und mittels 1,5 g/m² aufgesprühten Hot-melt-Klebers an der flüssigkeitsundurchlässigen Schicht **11** befestigt.

### Vergleichsbeispiel:

Die Ausgangsmaterialien stimmten im wesentlichen mit denen des Ausführungsbeispieles überein.
Als Flüssigkeitsverteitvlies wird das in DE 29723320 U1 beschriebene Material aus einer Polyethylen/Polyester-Mantel/Kern-Bikomponentenfaser des Fasertiters von 3,3 dtex, mit einer Flächenmasse von 30 g/m² und einer Dicke gemessen nach DIN 53855 T2 von 1,2 mm, was einer Dichte von 25 kg/m³ entspricht. Es entspricht dem Vliesstoff niederer Dichte des Ausführungsbeispieles, wird aber seiner veränderten Aufgabe entsprechend in anderer Weise eingesetzt.

Der Aufbau des Vergleichsbeispieles ist demnach wie folgt: Unter der körperseitige Abdeckung wird das Flüssigkeitsverteilvlies positioniert und mit 1,5 g/m² aufgesprühten Hot-melt-Klebers an der bekleidungsseitigen Abdeckung befestigt.

Darunter folgen eine erste und eine zweite Schicht des hydrophilen Meltblownvliesstoffes des Ausführungsbeispieles. Die Schichten werden ebenfalls mit 1,5 g/m² aufgesprühten Hot-melt-Klebers untereinander verbunden. Darunter wird als weitere Schicht eines hoch flüssigkeitsbindenden Materials in Form eines Tissue-Papieres eingelegt und mittels 1,5 g/m² aufgesprühten Hot-melt-Klebers an der bekleidungsseitigen Abdeckung befestigt.

### Vergleichsmessungen:

### Meßmethode zur Bestimmung der Rücknässung:

Es werden mittels einer Tropfpipette 5 ml "synthetic menstrual fluid"*) gleichmäßig über einen Zeitraum von 3 min. auf den zu testenden Artikel aufgebracht. Nach einer Einwirkzeit von 2 Minuten werden 10 Lagen Filterpapier Whatman # 4, deren Masse vorher ermittelt wurde, mit einem Durchmesser von 150 mm auf die Eintragsstelle aufgelegt und für 1 Minute mit einer Masse von 1,5 kg bei einem Durchmesser von 60 mm belastet. Über die Rückwägung des Filterpapieres und die Differenzbildung zwischen den Werten des trockenen und des beaufschlagten Filterpapieres wird der Rücknässungswert in Gramm ermittelt.
*) Anleitung zur Herstellung: Broschüre "Favor ® absorbent polymers", "Synthetic menstrual fluid - A substitute for menstrual fluids to characterize the properties of superabsorbent polymers in feminine hygiene applications" - Chemische Fabrik Stockhausen, D-Krefeld

### Ergebnisse:

| Merkmal | Einheit | Ausführungsbeispiel | Vergleichsbeispiel |
|---|---|---|---|
| Rücknässung | Gramm | 0,06 | 1,54 |

## Patentansprüche

1. Absorbierender Artikel mit einer Längausdehnung in x - Richtung, einer Querausdehnung und einer Dicke,
bestehend aus einer oberen, dem Körper zugewandten flüssigkeitsdurchlässigen Abdeckschicht,
aus einer unteren, der Bekleidung des Trägers zugewandten flüssigkeitsundurchlässigen Schicht
und einem, sich zwischen diesen Schichten befindlichen mehrschichtigen absorbierenden Kern,
welcher zumindest aus einer ersten Kernschicht aus hydrophilem synthetischen Mikrofaservlies besteht, welche von einem Vliesstoff niedriger Dichte umhüllt ist, und aus einer daran sich anschließenden zweiten Kernschicht aus hydrophilem synthetischen Mikrofaservlies,
welche in Kontakt ist mit einer weiteren Kernschicht aus hoch wasserspeicherndem Material
dadurch gekennzeichnet,
daß die Umhüllung **15** der ersten Kernschicht **13** an ihrer Oberseite ausgehend vom Schnittpunkt **9** der Längs- und Querachse eine Öffnung **6** aufweist,
daß an der Unterseite der ersten Kernschicht **13** zu beiden Seiten der Längsachse in x - Richtung ein längs verlaufender nicht umhüllter Bereich **12** verbleibt, welcher von der Umhüllung **15** begrenzt ist,
daß die erste Kernschicht **13** an deren Oberseite zumindest im Bereich der Öffnung **6** mit der flüssigkeitsdurchlässigen Abdeckschicht **10** fest verbunden ist,
daß die erste Kernschicht **13** an deren Unterseite zumindest am nicht umhüllten Bereich **12** mit der zweiten Kernschicht **14** fest verbunden ist,
und daß sich an die zweite Kernschicht **14** die weitere Kernschicht **16** aus hoch wasserspeichendem Matrial anschließt.

2. Absorbierender Artikel nach Anspruch 1,
dadurch gekennzeichnet,
daß die obere, dem Körper zugewandte flüssigkeitsdurchlässige Abdeckschicht **10** aus einem Vliesstoff besteht.

3. Absorbierender Artikel nach Anspruch 2,
dadurch gekennzeichnet,
daß der Vliesstoff perforiert ist.

4. Absorbierender Artikel nach Anspruch 2 und 3,
dadurch gekennzeichnet,
daß der Vliesstoff hydrophob ist.

5. Absorbierender Artikel nach Anspruch 1
dadurch gekennzeichnet,
daß die obere, dem Körper zugewandte flüssigkeitsdurchlässige Abdeckschicht **1** aus einer perforierten Folie besteht.

6. Absorbierender Artikel nach Anspruch 1,
dadurch gekennzeichnet,
daß das hydrophile synthetische Mikrofaserviles, der ersten Kernschicht **13** und der zweiten Kernschicht **14** ein Polypropylen-Meltblown-Vliesstoff ist.

7. Absorbierender Artikel nach Anspruch 1 und 6,
dadurch gekennzeichnet,
daß das hydrophile synthetische Mikrofaservlies, der ersten Kernschicht **13** und der zweiten Kernschicht **14** jeweils eine Flächenmasse von 10 bis 100 g/m² aufweist.

8. Absorbierender Artikel nach Anspruch 1,
dadurch gekennzeichnet,
daß das hydrophile synthetische Mikrofaservlies, der ersten Kernschicht **13** und das hydrophile synthetische Mikrofaservlies der zweiten Kernschicht **14** gleiche oder unterschiedliche Fasermassen aufweisen.

9. Absorbierender Artikel nach Anspruch 1,
dadurch gekennzeichnet,
daß das hydrophile synthetische Mikrofaservlies, der ersten Kernschicht **13** und das hydrophile synthetische Mikrofaservlies der zweiten Kernschicht **14** jeweils einlagig oder mehrlagig ist.

10. Absorbierender Artikel nach Anspruch 1,
dadurch gekennzeichnet,
daß die Umhüllung **15** aus einem Vliesstoff niedriger Dichte aus synthetischen Fasern besteht und eine Dichte, von 10 - 50 kg/m³, bei einer Flächenmasse von 25 bis 70 g/m² aufweist.

11. Absorbierender Artikel nach Anspruch 1,
dadurch gekennzeichnet,
daß die weitere Kernschicht **16** Zellulosefasern enthält.

12. Absorbierender Artikel nach den Ansprüchen 1 und 11,
dadurch gekennzeichnet,
daß die weitere Kernschicht **16** ein Tissue oder einen Airlaid-Vliesstoff enthält.

13. Absorbierender Artikel nach Anspruch 1,
dadurch gekennzeichnet,
daß die weitere Kernschicht **16** superabsorbierende Partikel und/oder superabsorbierende Fasern enthält.

14. Absorbierender Artikel nach Anspruch 1,
dadurch gekennzeichnet,
daß die weitere Kernschicht **16** ein Tissue oder einen Airlaid-Vliesstoff in Verbindung mit superabsorbierenden Partikeln und/oder superabsorbierenden Fasern enthält.

15. Absorbierender Artikel nach Anspruch 1,
dadurch gekennzeichnet,
daß die der Bekleidung des Trägers zugewandte flüssigkeitsundurchlässige Schicht **11** luftdicht ist.

16. Absorbierender Artikel nach Anspruch 1,
dadurch gekennzeichnet,
daß flüssigkeitsundurchlässige Schicht **11** wasserdampfdurchlässig ist.

17. Absorbierender Artikel nach Anspruch 1,
dadurch gekennzeichnet,
daß die feste Verbindung der ersten Kernschicht **13** mit der flüssigkeitsdurchlässigen Abdeckschicht **10** im Bereich der Öffnung **6** eine Hot-Melt-Verklebung ist.

18. Absorbierender Artikel nach Anspruch 1,
dadurch gekennzeichnet,
daß die erste Kernschicht **13** an ihrer Unterseite an der Stelle des nicht umhüllten Bereiches mit der zweiten Schicht mittels Hot-Melt-Verklebung fest verbunden ist.

19. Absorbierender Artikel nach Anspruch 1,
dadurch gekennzeichnet,
daß die Umhüllung **15** an den Übergängen zur Öffnung **6** an der Oberseite der ersten Kernschicht **13** und zum nicht umhüllten Bereich **12** an der Unterseite der ersten Kernschicht **13** einen Dichtegradienten aufweist.
